# EUROPEAN PATENT APPLICATION

(11) **EP 1 688 169 A1**
(43) Date of publication of application: **09.08.2006**
(21) Application number: 04819382.5
(22) Date of filing: 25.11.2004
(51) Int. Cl.: B01D 9/02, B01J 19/10, A61K 31/192, A61K 47/10

(54) **METHOD FOR PRODUCING FINE ORGANIC COMPOUND PARTICLES**

(30) Priority: 28.11.2003 JP 2003399617
(71) Applicant: MITSUBISHI CHEMICAL CORPORATION, Tokyo 108-0014 (JP)
(72) Inventor: SEKI, Hiroya, Yokohama-shi, Kanagawa 2278502 (JP); ASATANI, Haruki, Yokohama-shi, Kanagawa 2278502 (JP)
(74) Representative: HOFFMANN EITLE
(86) International application number: PCT/JP2004/017448
(87) International publication number: WO 2005/051511

(57) **Abstract**

There is provided a process for producing fine particles of an organic compound by a continuous-type poor solvent precipitation method which is capable of rapidly and uniformly dispersing an organic compound-containing solution in a large amount of a poor solvent, thereby more readily producing fine particles of the organic compound having a volume-average particle size of not more than 1 *µ*m. In the process for producing fine particles of an organic compound, the poor solvent in which the organic compound is hardly soluble, is continuously mixed with the organic compound-containing solution prepared by dissolving the organic compound in a good solvent which is miscible with the poor solvent and in which the organic compound is readily soluble, while irradiating an ultrasonic wave thereto.

## Description

### TECHNICAL FIELD

The present invention relates to a process for producing fine particles of an organic compound, and more particularly, to a process for producing fine particles of an organic compound, which process is capable of producing ultrafine particles of the organic compound which are hardly soluble in water and have a particle size of not more than 1 *µ*m, by utilizing a continuous-type poor solvent precipitation method.

### BACKGROUND ART

Particles of hardly water-soluble organic compounds, namely, those particles of organic compounds having a solubility in water of less than about 10 mg/mL, have been extensively used for producing various products such as medicines or drugs, inks, dyes, lubricants, insecticides, agricultural chemicals, fertilizers and cosmetics. In many cases, these products can be enhanced in quality by controlling a particle size of the particles contained therein.

For example, medicines or drugs, in particular, hardly water-soluble drugs exhibit an extremely low elution rate and, as a result, may fail to be sufficiently eluted out even after passing beyond an aimed in-vivo absorption site in some cases. Therefore, the hardly water-soluble drugs have problems such as deteriorated in-vivo utilization efficiency. In consequence, there has been proposed a method in which the drugs are finely divided into fine particles having a particle size of about sub-micron order to increase a specific surface area thereof and enhance a dissolution rate thereof, thereby improving bioavailability of the drugs (Japanese Patent Publication (KOKOKU) No. 60-24768(1985)). Further, there have been proposed methods of finely dividing the hardly water-soluble drugs by mechanical pulverization (Japanese Patent No. 2642486 and Japanese Patent Application Laid-Open (KOKAI) No. 4-295420(1992)).

In addition, as the method of preparing fine particles of hardly water-soluble drugs, there has been used a so-called poor solvent precipitation method in which after once dissolving the hardly water-soluble drugs in an organic solvent, etc., the resultant organic solvent solution of the drugs is mixed with a solvent in which the organic solvent is mutually dissolvable but the drugs are hardly soluble, i.e., a poor solvent for the drugs, to prepare such a mixed solution in which the drugs are present at a concentration exceeding a solubility thereof, thereby allowing the drugs to be precipitated in the form of particles. As such a method, there has been proposed a batch-type method for producing particles of drugs having a particle size of a sub-micron order by dissolving the drugs in an organic solvent in which water is mutually dissolvable, and then adding water as a poor solvent for the drugs to the resultant organic solvent solution while stirring, at a specific temperature and a specific velocity (Japanese Patent Application Laid-Open (KOKAI) No. 62-27032(1987)). Also, there has been proposed a batch-type method for producing fine particles of an organic compound having an average particle size of 400 nm to 2 *µ*m by dissolving the organic compound in a specific water-miscible first solvent, mixing the resultant solution with water as a second solvent to prepare a pre-suspension, and then applying an energy to the obtained pre-suspension to form the aimed fine particles (US Patent No. 6,607,784B2).

As the method for producing fine particles by utilizing the poor solvent precipitation method, there has also been proposed a continuous-type method of continuously adding and mixing a poor solvent and a drug-containing solution in a suspension of fine particles which are circulated by a pump, etc., to continuously discharge a part of the suspension of fine particles as a suspension product (US Patent Application 2003/0049323A1). In such poor solvent precipitation methods as described above, as pointed out in the respective patent documents, in order to produce particles having a narrow particle size distribution and a small average particle size, it is important to mix the drug-containing solution and the poor solvent with each other at a high speed.

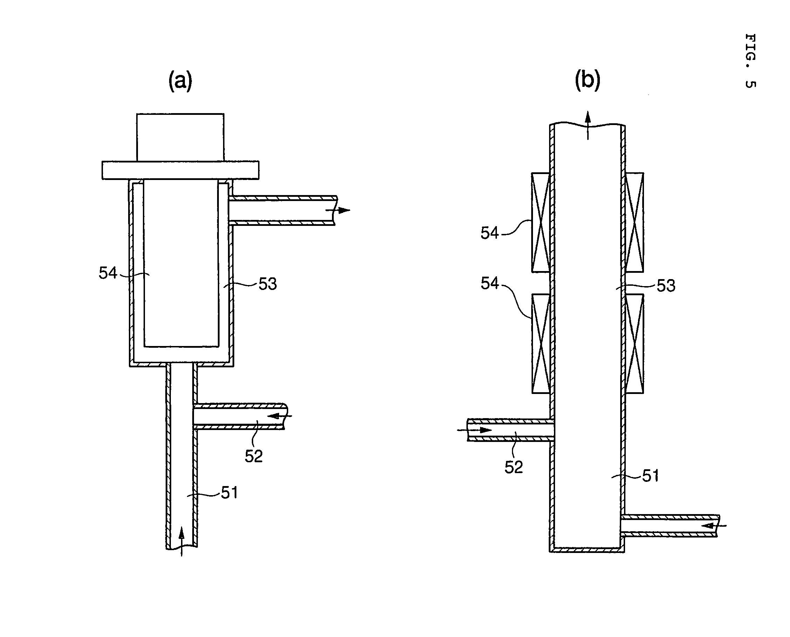

There is known for a long time a method in which two or more kinds of fluids are mixed with each other while flowing these fluids and simultaneously irradiating an ultrasonic wave thereto for the purpose of promoting mixing therebetween. Fig. 5 shows a vertical cross-sectional view of a conventional mixing apparatus using an ultrasonic wave as a means for mixing two kinds of fluids with each other. In Fig. 5, reference numeral (51) denotes a main flow path for flowing one of the fluids therethrough, reference numeral (52) is an introduction flow path for flowing the other of the fluids therethrough, reference numeral (53) is a mixing chamber, and reference numeral (54) is an ultrasonic horn or an ultrasonic oscillator for irradiating an ultrasonic wave to the fluid.

For example, the mixing apparatus shown in Fig. 5(a) is used for mixing water with an oil to produce an emulsion with a high efficiency (Masanori SHIMAKAWA, "Ultrasonic Technology" edited by Industrial Research Institute, p. 505 (1975)). Also, the mixing apparatus shown in Fig. 5(b) is used in a reaction precipitation method in which two kinds of fluids to be mixed are contacted and chemically reacted with each other to produce particles hardly soluble in the resultant mixed fluid. Specifically, the mixing apparatus is used in such a continuous-type method of continuously producing fine particles by irradiating an ultrasonic wave to a mixed fluid flowed therethrough to promote mixing between two kinds of fluids (US Patent No. 6,465,015B1). Meanwhile, the above method of producing fine particles is a method for producing fine particles in a non-agglomerated state upon production of the aimed product by chemical reaction of the raw compound, and, therefore, such a method is different from the poor solvent precipitation method.

In the above emulsion production method and the above method of producing fine particles, as respectively shown in Fig. 5, in order to contact the two kinds of fluids with each other, one of the fluids is introduced through an introduction flow path branched from a main flow path. The two kinds of fluids introduced through the main and introduction flow paths, respectively, are contacted with each other in the main flow path, and an ultrasonic wave is irradiated to the resultant mixed fluid. In these production methods, flow rates of the two fluids are generally substantially equal to each other.

### DISCLOSURE OF THE INVENTION

### Problem to be Solved by the Invention

However, in the above method of finely dividing drugs into particles by mechanical pulverization, there tend to be caused disadvantages such as low yield, occurrence of noise and generation of dusts, and further there tends to arise such a problem that the drugs are contaminated with pulverization media such as steel balls for impact pulverization. Also, in the batch-type poor solvent precipitation method, the particle size of the drugs varies depending upon introduction velocity of the poor solvent, stirring speed of the solution, etc., resulting in difficulty in scale-up of the process. As a result, it may be difficult to realize an industrial scale production of the aimed product.

On the other hand, in the above continuous-type poor solvent precipitation method, it is intended to promote mixing of the poor solvent with the drug-containing solution by turbulent energy of the fluids (Reynolds number: not less than 4000). In fact, in some cases, this method may fail to attain a high mixing velocity sufficient to obtain fine particles having a particle size of a sub-micron order. Further, as mentioned below, in the poor solvent precipitation method, the volume ratio of the poor solvent to the drug-containing solution is generally controlled to 10:1 to 100:1 in order to obtain sub-micron particles. Accordingly, even when irradiating an ultrasonic wave to these fluids, the fluid-contacting method as used in the above emulsion production method is insufficient to rapidly disperse a small amount of the drug-containing solution in a large amount of the poor solvent and obtain a uniform dispersion thereof.

The present invention has been made in view of the above conventional problems. An object of the present invention is to provide a process for producing fine particles of an organic compound, comprising utilizing a continuous-type poor solvent precipitation method which is capable of rapidly and uniformly dispersing an organic compound-containing solution in a large amount of a poor solvent and more readily producing fine particles of the organic compound having a particle size of not more than 1 *µ*m.

### Means for Solving Problems

As a result of the present inventors' earnest study for solving the above problems, it has been found that in the case where the poor solvent is mixed with the organic compound-containing solution while irradiating an ultrasonic wave thereto, the organic compound-containing solution can be rapidly and uniformly dispersed in the poor solvent, thereby more readily producing fine particles of the organic compound. The present invention has been attained on the basis of the finding.

That is, in an aspect of the present invention, there is provided a process for producing fine particles of an organic compound, comprising continuously mixing a poor solvent in which the organic compound is hardly soluble, with an organic compound-containing solution prepared by dissolving the organic compound in a good solvent which is miscible with the poor solvent and in which the organic compound is readily soluble,
wherein the poor solvent and the organic compound-containing solution are mixed with each other while irradiating an ultrasonic wave thereto.

In another aspect of the present invention, there is provided a process for producing fine particles of an organic compound, comprising continuously mixing a poor solvent in which the organic compound is hardly soluble, with an organic compound-containing solution prepared by dissolving the organic compound in a good solvent which is miscible with the poor solvent and in which the organic compound is readily soluble,
wherein the poor solvent and the organic compound-containing solution are mixed with each other by means of a coaxial mixer having a main flow path and an introduction flow path disposed along a centerline of the main flow path, the poor solvent is passed through the main flow path whereas the organic compound-containing solution is passed through the introduction flow path, and an ultrasonic wave is irradiated to a region where the poor solvent and the organic compound-containing solution are contacted with each other.

### EFFECT OF THE INVENTION

In the process for producing fine particles of an organic compound according to the present invention, since the poor solvent and the organic compound-containing solution are mixed with each other while irradiating an ultrasonic wave thereto, the organic compound-containing solution can be rapidly and uniformly dispersed in a large amount of the poor solvent, thereby enabling fine particles of the organic compound having a volume-average particle size of not more than 1 *µ*m, in particular, about 0.1 to 0.7 *µ*m to be continuously and readily produced.

### BRIEF DESCRIPTION OF DRAWINGS

Fig. 1 is a process flow diagram showing an entire construction of a process for producing fine particles according to an embodiment of the present invention.
Fig. 2 is a vertical cross-sectional view showing a structural example of essential parts of a coaxial mixer used in the process for producing fine particles according to the present invention.
Fig. 3 is a lateral cross-sectional view showing a shape of section of the coaxial mixer, taken in the direction perpendicular to centerlines of main and introduction flow paths thereof.
Fig. 4 is a vertical cross-sectional view showing another structural example of essential parts of the coaxial mixer.
Fig. 5 is a vertical cross-sectional view showing a conventional mixing apparatus using an ultrasonic wave as a means for mixing two kinds of fluids with each other.

### Explanation of Reference Numerals

1: Main flow path; 2: Introduction flow path; 3: Mixing chamber; 4a: Ultrasonic oscillator (ultrasonic horn); 4b: Ultrasonic oscillator; S: Mixing region; A: Mixing precipitation step; B: Dispersion/stabilization step; C: Solid-liquid separation step

### PREFERRED EMBODIMENTS FOR CARRYING OUT THE INVENTION

The preferred embodiment of the process for producing fine particles of an organic compound according to the present invention (hereinafter referred to merely as a "fine particle production process") is explained in detail below by referring to the accompanying drawings. Meanwhile, the preferred embodiment as well as constitutional elements involved therein as described hereinlater are only a typical example of the present invention and, therefore not intended to limit the scope of the present invention.

The fine particle production process according to the present invention is a so-called continuous-type poor solvent precipitation method wherein the poor solvent in which the organic compound is hardly soluble, is continuously mixed with the organic compound-containing solution prepared by dissolving the organic compound in the good solvent which is miscible with the poor solvent and in which the organic compound is readily soluble, thereby producing fine particles of the organic compound. In the present invention, the poor solvent and the organic compound-containing solution are rapidly and uniformly mixed with each other while irradiating an ultrasonic wave thereto.

The method of mixing the poor solvent with the organic compound-containing solution while irradiating an ultrasonic wave to the mixed solution thereof is not particularly limited as long as the ultrasonic wave is irradiated at the mixing stage including the stage of initiating the mixing by contacting the poor solvent with the organic compound-containing solution. The poor solvent and the organic compound-containing solution are preferably mixed with each other using a coaxial mixer having a main flow path and an introduction flow path disposed along a centerline of the main flow path in order to uniformly and efficiently conduct the mixing therebetween and produce finer particles. The poor solvent and the organic compound may be respectively supplied through either the main flow path or the introduction flow path as long as these components are intimately mixed with each other by controlling flow rates thereof, thereby producing the aimed fine particles. In order to uniformly and efficiently mix the poor solvent with the organic compound-containing solution and produce finer particles of the organic compound, it is preferred that the poor solvent is passed through the main flow path and the organic compound-containing solution is passed through the introduction flow path.

Also, for the purpose of obtaining the fine particles of a sub-micron order, upon mixing the poor solvent with the organic compound-containing solution, the volumetric flow rate ratio of the former to the latter is preferably adjusted to 10:1 to 100:1. Further, in order to obtain a suspension of the fine particles having a smaller particle size and a good dispersion stability by mixing the poor solvent with the organic compound-containing solution, a dispersion stabilizer is preferably added to either the poor solvent or the organic compound-containing solution. In addition, if required, the suspension of the fine particles which is obtained by mixing the poor solvent with the organic compound-containing solution, may be subjected to wet pulverization using a high-pressure homogenizer or an ultrasonic homogenizer.

Meanwhile, in the fine particle production process according to the present invention, the fine particles are obtained in the form of a dispersion in which the fine particles are dispersed in a mixture of the poor solvent and the good solvent. In the case where the fine particles of the organic compound are obtained in a solid state, the fine particle production process according to the present invention usually includes (A) a mixing/precipitation step of continuously mixing the poor solvent with the organic compound-containing solution to produce a suspension of the fine particles; (B) a dispersion/stabilization step of dispersing the fine particles contained in the suspension to stabilize the suspension; and (C) a solid-liquid separation step of separating the stabilized suspension of the fine particles into the solid fine particles and the solvent, wherein in the mixing/precipitation step (A), the poor solvent and the organic compound-containing solution are mixed with each other while irradiating an ultrasonic wave thereto.

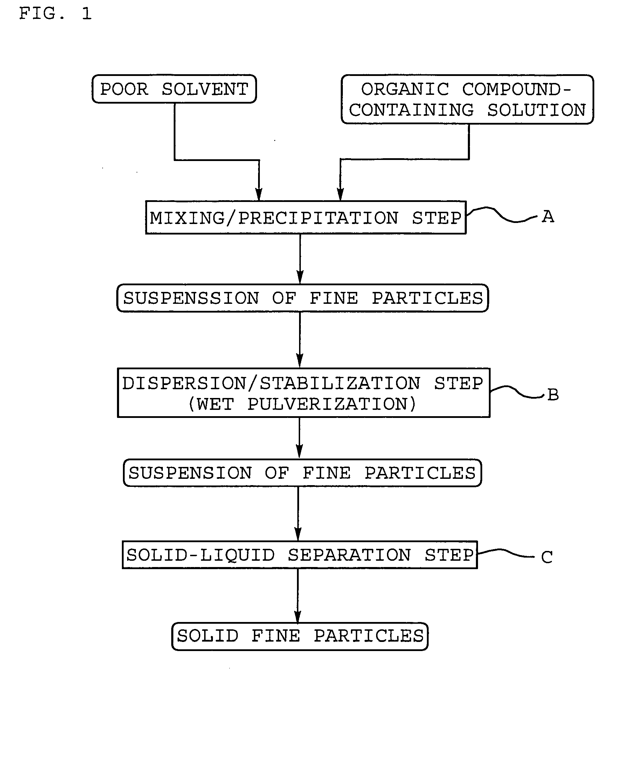

The preferred embodiment of the present invention is described in detail below by referring to the accompanying drawings. As shown in Fig. 1, for obtaining the fine particles in a solid state, the fine particle production process according to the present invention usually includes (A) a mixing/precipitation step of continuously mixing the poor solvent with the organic compound-containing solution to produce a suspension of the fine particles; (B) a dispersion/stabilization step of dispersing the fine particles contained in the suspension to stabilize the suspension; and (C) a solid-liquid separation step of separating the stabilized suspension of the fine particles into the solid fine particles and the solvent. Meanwhile, the construction of each apparatus used in the present invention is explained together with detailed contents of the above respective steps.

In the above mixing/precipitation step (A), although not shown in the figures, the organic compound-containing solution prepared by dissolving the organic compound in the solvent is usually supplied from an organic compound-containing solution storage tank, whereas the poor solvent is usually supplied from a poor solvent storage tank. In this case, the organic compound-containing solution and the poor solvent are delivered by a feed pump, and the temperature thereof is suitably controlled by a heat exchanger disposed in the course of the flow path therefor. Alternatively, the temperatures of the organic compound-containing solution and the poor solvent may be controlled by an adequate temperature controlling means respectively provided in the organic compound-containing solution storage tank and the poor solvent storage tank in place of using the heat exchanger.

The organic compound to be treated according to the present invention mainly includes those hardly soluble in water. That is, in this case, the poor solvent for the organic compound is water, and the good solvent therefor is a so-called organic solvent. However, the present invention can also be applied to water-soluble organic compounds by using suitable solvents in combination with the organic compounds. The solvent also involves supercritical fluids.

In the present invention, the hardly water-soluble organic compound means such an organic compound whose solubility in water, in particular, 20°C water is usually not more than 10 mg/mL. The solubility in 20°C water of the hardly water-soluble organic compound to be treated by the present invention is preferably not more than 5 mg/mL, more preferably not more than 1 mg/mL. The organic compound having a lower solubility in water is preferably used, in particular, in the case where water is used as the poor solvent, since fine particles of the organic compound can be produced in a more advantageous manner. In particular, the medicines mean such drugs exhibiting an insufficient systemic absorption when formulated into solid preparations by ordinary formulation methods. Examples of the drugs may include coronary vasodilators such as nifedipine and nicardipine, steroid anti-inflammatory drugs such as cortisone and petemethasone, non-steroid anti-inflammatory drugs such as indomethacin and naproxen, anti-psychoneurosis drugs such as phenytoin and phenacemide, cardiac drugs such as ubidecareneone, chemotherapeutic drugs such as griseofulvin, antihistaminic drugs such as mequitazine or the like.

The poor solvent may be alone or in the form of a mixed solvent of two or more kinds of solvents in which the organic compound is hardly soluble. That is, the organic compound has a solubility in the poor solvent of usually not more than 10 mg/mL, and water is preferably used as the poor solvent. However, when a supercritical CO₂ fluid is used in the rear-stage solid-liquid separation step (C), as the poor solvent, there may be used a solvent or a mixed solvent miscible with the supercritical CO₂ fluid, preferably heptane or hexane.

As the good solvent, there may be used those solvents in which the hardly water-soluble organic compound can be dissolved and which is miscible with the poor solvent. These good solvents may be used alone or in the form of a mixed solvent of two or more thereof. Among these good solvents, preferred are organic solvents which can be readily removed in the rear-stage step. Examples of such organic solvents may include methanol, ethanol, isopropyl alcohol, 1-butanol, n-methyl pyrrolidone, acetone, tetrahydrofuran, dimethyl formamide, ethylenediamine, acetonitrile, methyl ethyl ketone, dimethyl sulfoxide and dichloromethane. However, when the supercritical CO₂ fluid is used in the rear-stage solid-liquid separation step (C), as the good solvent, there may be used solvents miscible with the supercritical CO₂ fluid.

Meanwhile, in the present invention, as the good solvent which is miscible with the poor solvent and in which the organic compound can be readily dissolved, there are preferably selected such solvents which are mutually dissolvable in the poor solvent, namely, solvents which are free from separation into two liquid-liquid phases at the temperature and mixing ratio used when being mixed with the poor solvent. The solubility of the organic compound in the good solvent is usually not less than 10 mg/mL, preferably not less than 20 mg/mL. The upper limit of the solubility of the organic compound in the good solvent is not particularly limited, and is usually about 200 mg/mL. The concentration of the organic compound in the organic compound-containing solution may be controlled so as not to exceed a saturation solubility thereof at room temperature, and is preferably 50 to 100% of the saturation solubility.

In the mixing/precipitation step (A), the poor solvent and the organic compound-containing solution are mixed with each other to produce a suspension of the fine particles of the organic compound. In order to obtain a suspension of the fine particles having a smaller particle size and an excellent dispersion stability in the mixing/precipitation step (A), at least one kind of a dispersion stabilizer may be added to the organic compound-containing solution, the poor solvent or both thereof.

The kind of the dispersion stabilizer to be added varies depending upon kinds of the organic compound and solvent used, and usually may be selected from nonionic, anionic and cationic surfactants, polymers, phospholipids, etc. Examples of the especially preferred dispersion stabilizer may include polyoxyethylene sorbitan fatty esters (tradename: "TWEEN"), sucrose fatty esters, sorbitan fatty esters (tradename: "SPAN"), polyoxyethylene fatty ethers, aerosols (AOT), sodium laurylsulfate, sodium deoxycholate, polyvinyl pyrrolidone, polyvinyl alcohol, polyoxyethylene/polyoxypropylene glycol (tradename: "PLURONIC"), polyethyleneglycol, polyoxyethylene castor oil, hydroxypropyl cellulose, dextran, gelatin, casein, lecithin, etc. The concentration of the dispersion stabilizer in the organic compound-containing solution and/or the poor solvent is preferably controlled such that the weight ratio of the dispersion stabilizer to the organic compound in the suspension of the fine particles as produced is preferably in the range of 0.01 to 10.

Upon mixing the poor solvent with the organic compound-containing solution in the mixing/precipitation step (A), the volumetric flow rate ratio therebetween may be experimentally determined depending upon the organic compound and the solvent used. In general, for the purpose of obtaining fine particles of a sub-micron order, the volumetric flow rate ratio of the poor solvent to the organic compound-containing solution is usually 1:1 to 100:1, preferably 5:1 to 100:1, more preferably 10:1 to 100:1.

In the mixing/precipitation step (A), as described above, the volumetric flow rate of the poor solvent is generally greatly larger than that of the organic compound-containing solution. Therefore, when the temperature of the poor solvent is controlled using a heat exchanger or a temperature-controlling means in the storage tank, the temperature of the resultant suspension of the fine particles becomes substantially identical to that of the poor solvent. Accordingly, the temperature of the poor solvent may be adjusted to such a temperature suitable for precipitating the fine particles. On the other hand, the temperature of the organic compound-containing solution may be adjusted to the same temperature as that of the poor solvent or the temperature which is free from precipitation of particles of the organic compound in a conduit disposed before the position where the organic compound-containing solution is introduced into the mixing/precipitation step (A). Meanwhile, the temperature suitable for precipitation of the fine particles varies depending upon the organic compound to be treated, and is usually 0 to 90°C, preferably 0 to 50°C.

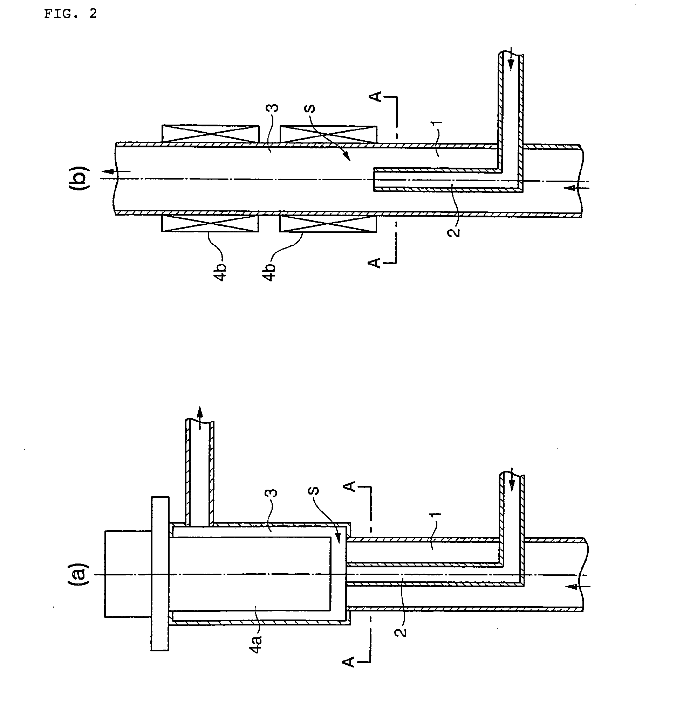

In the preferred embodiment of the present invention, upon continuously mixing the poor solvent with the organic compound-containing solution in the mixing/precipitation step (A), as a means for mixing the poor solvent with the organic compound-containing solution, there is used a coaxial mixer having a main flow path (1) and an introduction flow path (2) disposed along a centerline of the main flow path, as shown in Fig. 2. The poor solvent is passed through the main flow path (1), whereas the organic compound-containing solution is passed through the introduction flow path (2), and further the poor solvent and the organic compound-containing solution are mixed with each other while irradiating an ultrasonic wave to the mixed fluid thereof.

More specifically, in the present invention, it is required to initiate the irradiation of an ultrasonic wave not after initiating the mixing between the poor solvent and the organic compound-containing solution but at latest at the time at which the mixing between the poor solvent and the organic compound-containing solution is initiated by contact therebetween. Usually, the ultrasonic wave is continuously irradiated over a whole period of from the time at which the poor solvent is contacted with the organic compound-containing solution to initiate the mixing therebetween to the time at which the mixing by contact therebetween is substantially terminated. In other words, the ultrasonic wave is irradiated to a region where the poor solvent and the organic compound-containing solution are contacted with each other. With the above irradiation of an ultrasonic wave, in the present invention, the poor solvent and the organic compound-containing solution can be rapidly and uniformly mixed with each other, thereby obtaining a suspension of the fine particles and further producing the aimed fine particles of the organic compound in an efficient manner.

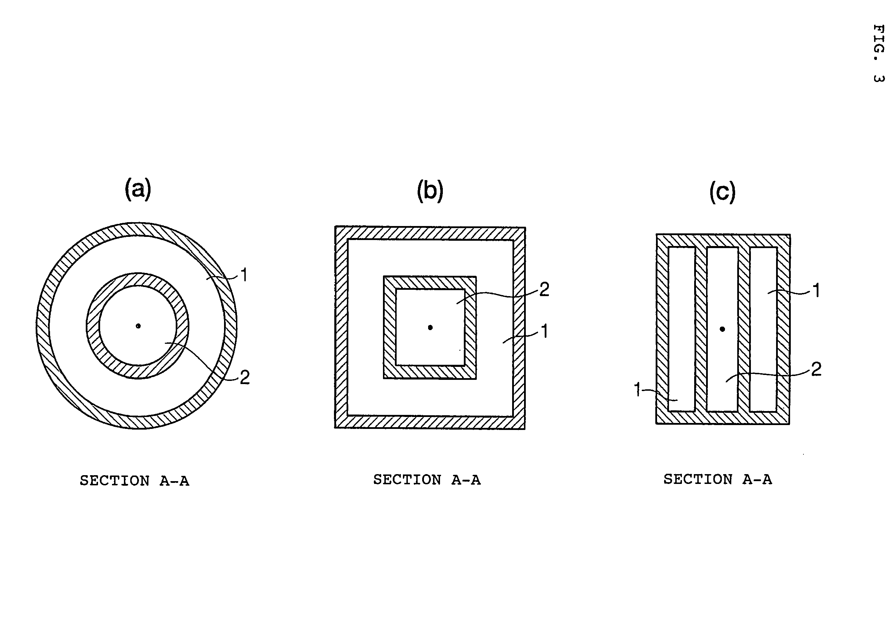

As shown in Fig. 2, the above coaxial mixer may be in the form of a fluid mixer for mixing two fluids with each other and has, for example, a double tube structure in which a tip end portion of the introduction flow path (2) is inserted to an inside of the main flow path (1) along a centerline of the main flow path. The section of each of the main flow path (1) and the introduction flow path (2) means a plane perpendicular to the direction of fluid flow in the respective flow paths. The shape of the section of the respective flow paths (section taken along the line A-A in Figs. 2(a) and 2(b)) may be, for example, a circular shape as shown in Fig. 3(a) or a rectangular shape as shown in Fig. 3(b). In addition, the sectional shape may also be designed into such a structure as shown in Fig. 3(c) in which an inside of the main flow path (1) is divided by the introduction flow path (2).

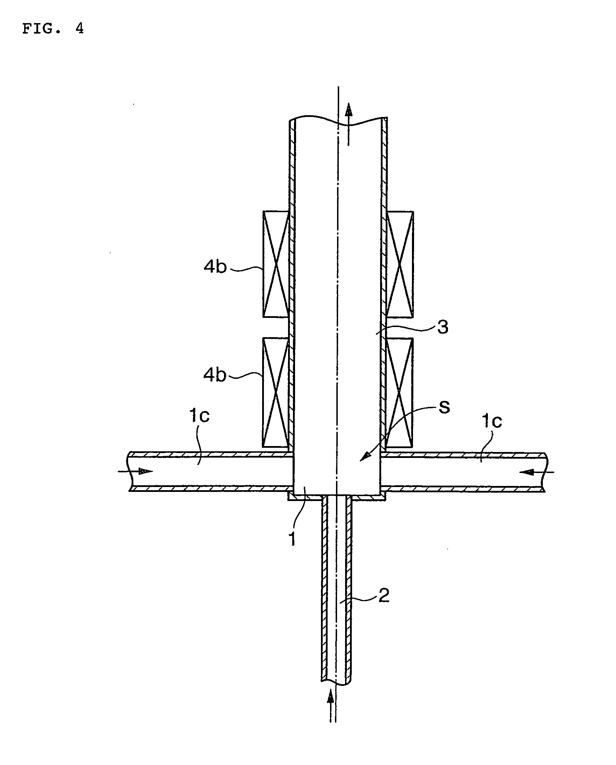

Further, the mutual arrangement of the main flow path (1) and the introduction flow path (2) in the coaxial mixer may be of such a structure in which the introduction flow path (2) is disposed along a centerline of the main flow path (1) and an outlet port of the introduction flow path (2) is located at a central position of the main flow path (1). Alternatively, the coaxial mixer may also have, for example, such a structure as shown in Fig. 4. The coaxial mixer shown in Fig. 4 includes a main flow path (1) into which a fluid is introduced from right and left feed flow paths (1c) communicated with a base end portion of the main flow path (1), and an introduction flow path (2) connected to the base end portion along a centerline of the main flow path.

In the above coaxial mixer, the poor solvent and the organic compound-containing solution are preferably fed through the main flow path (1) and the introduction flow path (2), respectively. The fluid flowing through the main flow path (1) may be in the form of either a laminar flow or a turbulent flow, whereas the fluid flowing through the introduction flow path (2) may also be in the form of either a laminar flow or a turbulent flow. Meanwhile, the chain lines shown in Figs. 2 and 4 and the dot shown in Fig. 3 indicate a center of tip end portions of the main flow path (1) and the introduction flow path (2).

In the present invention, as described above, the poor solvent and the organic compound-containing solution are mixed with each other while irradiating an ultrasonic wave thereto. More specifically, in order to irradiate an ultrasonic wave to the region where the poor solvent and the organic compound-containing solution are contacted with each other, the coaxial mixer is equipped with ultrasonic oscillators (4a) and (4b). The region of contact between the poor solvent and the organic compound-containing solution means whole points where the poor solvent and the organic compound-containing solution are contacted with each other, and substantially indicates a mixing region (S) where the poor solvent and the organic compound-containing solution are mixed with each other. Therefore, in the coaxial mixer, an ultrasonic oscillator (4a) or (4b) may be disposed at a position capable of irradiating an ultrasonic wave to the mixing region (S).

More specifically, for example, in the coaxial mixer shown in Fig. 2(a), the tip end of the main flow path (1) is connected to a mixing chamber (3) having a larger sectional area than that of the main flow path, and an outlet port of a tip end of the introduction flow path (2) is positioned at such a height level as to come into a substantially flush alignment with the tip end of the main flow path (1). Further, the ultrasonic oscillator (4a) for irradiating an ultrasonic wave is inserted into the mixing chamber (3) where the contact between the poor solvent and the organic compound-containing solution is substantially conducted. Also, in the coaxial mixer shown in Fig. 2(b), a tip end portion of the main flow path (1) which corresponds to a portion located on a forward (downstream) side of the outlet port of the tip end of the introduction flow path (2) forms the mixing chamber (3), and the ultrasonic oscillators (4b) for irradiating an ultrasonic wave are mounted on an outer periphery of the tip end portion of the main flow path (1) which forms the mixing chamber (3).

In the mixing/precipitation step (A), the frequency of the ultrasonic wave irradiated is usually 10 kHz to 2 MHz, preferably 10 to 100 kHz, more preferably 15 to 30 kHz. The time and intensity of the ultrasonic wave irradiated upon mixing two fluids with each other may be experimentally determined. In general, the longer the ultrasonic wave irradiating time and the stronger the intensity of the ultrasonic wave irradiated, the finer the resultant particles become.

The ultrasonic wave irradiating time may be calculated from a total flow rate of the two fluids and a volume of the region to be irradiated with the ultrasonic wave (i.e., mixing region (S)) as a residence time [(volume of mixing region) ÷ (volumetric flow rate)] of the mixed fluid in the mixing region (S). The ultrasonic wave irradiating time is preferably not less than 0.01 sec, more preferably not less than 0.1 sec. The upper limit of the ultrasonic wave irradiating time is not particularly limited, and is usually not more than 5 sec. However, in some cases, the ultrasonic wave irradiating time of not more than about 3 sec may be satisfactory depending upon kinds of the fluids to be irradiated with an ultrasonic wave. The intensity of the ultrasonic wave irradiated is preferably not less than 5 W/cm², more preferably not less than 10 W/cm² per sectional area of the flow path. The upper limit of the intensity of the ultrasonic wave irradiated is not particularly limited, and is usually 1000 W/cm². However, since a too high intensity of the ultrasonic wave irradiated increases the temperature of the mixed fluid, the upper limit of the intensity of the ultrasonic wave irradiated is preferably 500 W/cm².

Further, in the mixing/precipitation step (A), in order to promote the mixing effect by the irradiation of ultrasonic wave, a back pressure may be applied to the mixing region (S) where the poor solvent and the organic compound-containing solution are mixed with each other, namely, to the mixing chamber (3), thereby increasing a pressure applied to the fluids in the mixing region (S) and enhancing a cavitation effect therein. In the case where there is such a risk that the temperature of the mixing region (S) is undesirably increased by the irradiation of ultrasonic wave, a temperature-controlling means is preferably provided in the mixing chamber (3). The temperature-controlling means is preferably in the form of a jacketed water cooling-type cooler. In this case, the temperature of a cooling water may be adjusted to the temperature suitable for precipitation of the particles as described above. Thus, by irradiating an ultrasonic wave to the mixing chamber (3) where the poor solvent and the organic compound-containing solution are mixed with each other, i.e., the mixing region (S) as the region where the poor solvent and the organic compound-containing solution are contacted with each other, the mixing between the poor solvent and the organic compound-containing solution can be more efficiently promoted.

The suspension of the fine particles obtained in the above mixing/precipitation step (A), namely, the suspension containing the precipitated fine particles dispersed in the solvent, is immediately supplied to the dispersion/stabilization step (B), if required, to further disperse and stabilize the suspension. The dispersion/stabilization step (B) is conducted by a wet pulverization method using no pulverizing media such as steel balls for impact pulverization to pulverize and disperse the fine particles in the suspension, thereby further reducing the particle size of the fine particles. As the wet pulverization method using no pulverizing media, there may be used such a method of breaking and pulverizing agglomerated particles by kinetic energy of the fluids and impact energy due to the cavitation effect using an ultrasonic homogenizer, a high-pressure homogenizer (e.g., homogenizers known under the tradenames "MICRO-FLUIDIZER" manufactured by MFI Corporation (USA), "ALTIMIZER" manufactured by Sugino Machines Co., Ltd., and "NANOMIZER" manufactured by Yoshida Kikai Kogyo Co., Ltd.), a piston gap-type homogenizer (e.g., homogenizer manufactured by APV Gaulin GmbH), etc.

As the above ultrasonic homogenizer, there may be used such ultrasonic homogenizers having an oscillation frequency of 10 kHz to 2 MHz, preferably 10 to 100 kHz, more preferably 15 to 30 kHz. In the high-pressure homogenizer, a pressure of the fluid to be fed therethrough is preferably adjusted to 50 to 300 MPa. In these wet pulverization methods, the suspension of the fine particles is preferably cooled to prevent temperature rise thereof. Also, in the dispersion/stabilization step (B), the wet pulverization is preferably conducted by a continuous method (flow method). In addition, in the case where the use of only one wet pulverizer is insufficient, there may be used a required number of wet pulverizers connected in series to each other.

The thus obtained suspension of the fine particles may be subjected to adequate treatments such as adjustment of solid content therein, addition of additives thereto and sterilization treatment, and then may be directly used as a final product, e.g., as an injection preparation or drug without subjecting the suspension to the subsequent solid-liquid separation step (C). The solid content in the suspension may be controlled by methods known in the art such as distillation and membrane separation, thereby removing the poor solvent together with a part of the good solvent. However, in particular, when the solid content in the suspension is controlled by the distillation procedure, etc., the particles in the suspension tend to be agglomerated together. Therefore, the obtained suspension may be subjected again to the dispersion/stabilization procedure. For the purpose of producing oral preparations of drugs, the suspension of the fine particles whose solid content has been properly adjusted may be mixed with a hydrophilic base material such as sugars, and then immediately granulated by fluidized bed coating methods known in the art. Further, the suspension of the fine particles may be fed to the above solid-liquid separation step (C) and subjected to solid-liquid separation to obtain solid fine particles.

In the solid-liquid separation step (C), as the method of separating the suspension of the fine particles into the solid fine particles and the solvent, there may be used methods known in the art such as filtration method, e.g., filtration under pressure, filtration under reduced pressure and centrifugal filtration, spray-drying method, freeze-drying method, etc. The poor solvent and the good solvent which are further separated in the solid-liquid separation step (C) may be respectively recycled and reused as the good solvent for initially preparing the organic compound-containing solution and as the poor solvent to be mixed with the organic compound-containing solution.

Meanwhile, when the poor solvent and the good solvent used in the present invention are solvents miscible with a supercritical CO₂ fluid, the suspension of the fine particles may be contacted with the supercritical CO₂ fluid in the solid-liquid separation step (C), whereby the fine particles of the organic compound can be separated by filtration from the supercritical CO₂ fluid while extracting the poor solvent and the good solvent into the supercritical CO₂ fluid. Since the use of the supercritical CO₂ fluid can reduce the viscosity of fluids to be treated, the filtration procedure for separating the fine particles of the organic compound from the suspension can be further facilitated. In addition, the thus extracted poor solvent and good solvent may be separated, recovered and reused similarly to the above-described cases, and the CO₂ used for the above extraction may also be reused.

The order of operations of the dispersion/stabilization step (B) and the solid-liquid separation step (C) is optional. For example, the suspension of the fine particles obtained in the mixing/precipitation step (A) may be first fed to the solid-liquid separation step (C) to remove the good solvent and the poor solvent therefrom by suitable methods such as filtration, thereby obtaining solid fine particles. Thereafter, the resultant solid fine particles may be dispersed again in the poor solvent, and then the obtained suspension of the fine particles may be fed to the dispersion/stabilization step (B). In such a case, the poor solvent used for re-dispersing the solid fine particles may not be identical to the poor solvent used in the mixing/precipitation step (A), and the concentration of the fine particles in the dispersion may also be optionally adjusted. Further, upon the re-dispersing procedure, an additional amount of the dispersion stabilizer may be added to the dispersion.

In accordance with the preferred embodiment of the process for producing fine particles according to the present invention, as the means for mixing the poor solvent with the organic compound-containing solution, there is used the coaxial mixer having the above specific structure, and ultrasonic wave is irradiated to the region where the poor solvent and the organic compound-containing solution are contacted with each other, thereby enabling the organic compound-containing solution to be rapidly and uniformly dispersed in a large amount of the poor solvent. As a result, it is possible to produce the fine particles of the organic compound having a volume-average particle size of not more than 1 *µ*m, in particular, 0.1 to 0.7 *µ*m, in a continuous facilitated manner.

### EXAMPLES:

### Examples 1 to 5:

In the mixing/precipitation step (A), using a coaxial mixer with the structure as shown in Fig. 2(a), the poor solvent and the organic compound-containing solution were continuously mixed with each other to produce a suspension containing fine particles of the organic compound. The coaxial mixer had such a structure including a main flow path (1) constituted from a stainless steel cylindrical tube having an inner diameter of 7 mm, an introduction flow path (2) disposed within the main flow path (1) which was constituted from a stainless steel cylindrical tube having an outer diameter of 1.6 mm and an inner diameter of 0.8 mm, a large-diameter mixing chamber (3), and an ultrasonic homogenizer ("US-150T" (tradename) manufactured by Nippon Seiki Seisakusho Co., Ltd.; frequency: 20 kHz; output power: 150 W) equipped with an ultrasonic oscillator (4a) (ultrasonic horn having a diameter of 20 mm) which was inserted into the mixing chamber (3) so as to locate 5 mm just above an outlet port of the introduction flow path (2). In the coaxial mixer, the poor solvent supplied through the main flow path (1) and the organic compound-containing solution supplied through the introduction flow path (2) were contacted and mixed with each other in the mixing chamber (3) while irradiating an ultrasonic wave thereto, thereby obtaining fine particles of the organic compound.

As the organic compound-containing solution, there was used a drug-containing solution prepared by dissolving naproxen which was hardly soluble in water and had a solubility in water of about 20 *µ*g/mL, in ethanol (purity: 99.5%) as a good solvent therefor. Upon preparing the drug-containing solution, polyoxyethylene sorbitan monooleate "Tween 80 (tradename)" and sorbitan monostearate "SPAN 60 (tradename)" as stabilizers were previously dissolved in the ethanol. As the poor solvent, there was used desalted water. Using a metering pump, the above poor solvent and drug-containing solution were fed to the coaxial mixer. In this case, the temperature of the poor solvent was controlled to a predetermined value by passing through a heat exchanger before being fed to the coaxial mixer. Further, a jacketed cooling apparatus was disposed in the mixing chamber of the coaxial mixer, and used to control a temperature of a mixed solution of the poor solvent and the drug-containing solution to the same temperature as that of the poor solvent.

Upon mixing the poor solvent with the drug-containing solution in the mixing/precipitation step (A), an ultrasonic wave was irradiated to the mixed solution in the mixing chamber (3) of the coaxial mixer for about 0.7 sec using an ultrasonic homogenizer ("US-150T" (tradename) manufactured by Nippon Seiki Seisakusho Co., Ltd.; frequency: 20 kHz; output power: 150 W), thereby obtaining about 200 mL of a suspension of fine particles in the mixing chamber (3). Then, the resultant suspension was fed to the dispersion/stabilization step (B) for uniforming the dispersion thereof in which the suspension of fine particles was subjected to wet pulverization treatment for 30 min using the same ultrasonic homogenizer as used above. Upon the wet pulverization, the temperature of the suspension of fine particles was adjusted to the same temperature as used upon precipitating the particles. Further, before and after conducting the wet pulverization using the ultrasonic homogenizer, the median diameter of the suspension of fine particles (on the basis of volume) was measured using a laser diffraction particle size distribution meter ("SALD2000J" (trade name) manufactured by Shimadzu Seisakusho Co., Ltd.). The results of Examples 1 to 5 are shown in Table 1.

In Examples 1 to 4, the flow rate ratio of the poor solvent to the drug-containing solution in the mixing precipitation step (A) was controlled to 39:1, whereas in Examples 2, 3 and 5, the flow rate ratio of the poor solvent to the drug-containing solution was controlled to 20:1. When the flow rate of the poor solvent in the mixing/precipitation step (A) was reduced, the particle size of the resultant particles was increased. From the results, it was confirmed that in order to obtain finer particles, it was required to increase the flow rate of the poor solvent to a certain extent as compared to the flow rate of the drug-containing solution. In addition, in Example 2, the temperature of the mixing chamber (3) as the mixing region (S) where the poor solvent and the drug-containing solution were mixed with each other, was controlled to 5°C, whereas in Example 3, the temperature of the mixing chamber (3) was controlled to 20°C. From the above results, it was confirmed that the control of the temperature of the mixing region (S) in the coaxial mixer was effective for controlling the particle size of the obtained particles.

**Table 1**

| | Examples | | | | |
|---|---|---|---|---|---|
| | 1 | 2 | 3 | 4 | 5 |
| Naproxen (wt%) | 2.5 | 2.5 | 2.5 | 3.75 | 3.75 |
| Tween 80 (wt%) | 1.0 | 1.0 | 1.0 | 1.5 | 1.5 |
| SPAN 60 (wt%) | 1.5 | 1.5 | 1.5 | 2.25 | 2.25 |
| Precipitation temperature in mixing region (°C) | 5 | 5 | 20 | 5 | 5 |
| Solution flow rate (mL/min) | 2 | 3.9 | 3.9 | 2 | 3.9 |
| Poor solvent flow rate (mL/min) | 78 | 78 | 78 | 78 | 78 |
| Particle size 1 (*µ*m) | 0.76 | 0.95 | 1.04 | 0.78 | 0.96 |
| Particle size 2 (*µ*m) | 0.53 | 0.60 | 0.77 | 0.56 | 0.57 |

| | | | | | |
|---|---|---|---|---|---|
| Note) Particle size 1: Particle size before pulverization treatment using an ultrasonic homogenizer | | | | | |
| Particle size 2: Particle size after pulverization treatment using an ultrasonic homogenizer | | | | | |

### Comparative Example 1:

The same procedure as defined in Example 4 was conducted except that there was used a mixing apparatus as shown in Fig. 5(a), and the poor solvent was fed through a main flow path (51), whereas the drug-containing solution (organic compound-containing solution) was fed through an introduction flow path (52), thereby continuously producing a suspension of fine particles. As a result, it was confirmed that the resultant fine particles had an average particle size of 1.3 *µ*m before pulverization treatment using an ultrasonic homogenizer, and 0.65 *µ*m after the pulverization treatment which values both were larger than the corresponding average particle size values of the fine particles obtained in Example 4.

### Comparative Example 2:

A suspension of fine particles was prepared by using a coaxial mixer with the same double tube structure as used in the above Examples and using the same poor solvent and drug-containing solution (organic compound-containing solution) as used in Example 4. More specifically, the same procedure as defined in Example 4 was conducted except that the coaxial mixer had such a structure including a main flow path constituted from a stainless steel cylindrical tube having an inner diameter of 2.16 mm and an introduction flow path constituted from a stainless steel cylindrical tube having an outer diameter of 1.6 mm and an inner diameter of 0.8 mm; desalted water maintained at 5°C was fed through the main flow path at a flow rate of 670 mL/min (Reynolds number: about 7000), whereas the drug-containing solution was fed through the introduction flow path at a flow rate of 33.5 mL/min; and no ultrasonic wave was irradiated in the mixing chamber. About 200 mL of the resultant suspension of fine particles was subjected to wet pulverization for 30 min using the ultrasonic homogenizer by the same method as defined in Example 4. The particles contained in the suspension has a particle size of 5.5 *µ*m and 4.4 *µ*m, respectively, before and after the wet pulverization using the ultrasonic homogenizer. From the above results, it was confirmed that when the mixing was conducted only by turbulent flow without using an ultrasonic wave, it was difficult to produce fine particles having a particle size of less than 1 *µ*m even though the suspension was subjected to wet pulverization after production of the fine particles.

### Example 6:

Indomethacin (solubility in water: not more than 1 mg/mL) as a hardly water-soluble organic compound was dissolved in ethanol (purity: 99.5%) as a good solvent therefor to prepare a drug-containing solution having a drug concentration of 10 mg/mL. As a poor solvent, there was used desalted water in which 0.625 mg/mL of sodium laurylsulfate was dissolved. Using the same coaxial mixer with a double tube structure as used in Examples 1 to 5, the poor solvent was supplied through the main flow path (1) at a flow rate of 76 mL/min, and the drug-containing solution was supplied through the introduction flow path (2) at a flow rate of 2 mL/min, and an ultrasonic wave was irradiated to the region where the two fluids were contacted with each other, at a frequency of 20 kHz and an output power of 150W and at room temperature (about 25°C), thereby producing a suspension of fine particles. The thus produced suspension of fine particles was immediately measured using a dynamic light-scattering particle size distribution meter ("HPP5001" (tradename) manufactured by Malvern Inc.). As a result, it was confirmed that a volume-average particle size of the resultant particles was 0.81 *µ*m. Further, 80 mL of the suspension of fine particles was pulverized for 30 min using an ultrasonic homogenizer. As a result, it was confirmed that a volume-average particle size of the thus pulverized particles contained in the suspension was 0.46 *µ*m.

## Claims

1. A process for producing fine particles of an organic compound, comprising continuously mixing a poor solvent in which the organic compound is hardly soluble, with an organic compound-containing solution prepared by dissolving the organic compound in a good solvent which is miscible with the poor solvent and in which the organic compound is readily soluble,
wherein the poor solvent and the organic compound-containing solution are mixed with each other while irradiating an ultrasonic wave thereto.

2. A process according to claim 1, wherein the poor solvent and the organic compound-containing solution are mixed with each other by means of a coaxial mixer having a main flow path and an introduction flow path disposed along a centerline of the main flow path.

3. A process according to claim 1 or 2, wherein the poor solvent is passed through the main flow path, and the organic compound-containing solution is passed through the introduction flow path.

4. A process according to any one of claims 1 to 3, wherein the organic compound has a solubility in water of not more than 5 mg/mL as measured at 20°C, and the poor solvent is water.

5. A process according to any one of claims 1 to 4, wherein the good solvent is an organic solvent having a solubility in the organic solvent of not less than 10 mg/mL.

6. A process according to any one of claims 1 to 5, wherein either the poor solvent or the organic compound-containing solution contains a dispersion stabilizer.

7. A process according to any one of claims 1 to 6, wherein upon mixing the poor solvent with the organic compound-containing solution, a volumetric flow rate ratio of the poor solvent to the organic compound-containing solution is controlled to 10:1 to 100:1.

8. A process according to any one of claims 1 to 7, wherein a suspension of fine particles which is obtained by mixing the poor solvent with the organic compound-containing solution is subjected to wet pulverization.

9. A process according to any one of claims 1 to 8, comprising:
(A) a mixing/precipitation step of continuously mixing the poor solvent with the organic compound-containing solution to produce a suspension of fine particles;
(B) a dispersion/stabilization step of dispersing the fine particles contained in the suspension to stabilize the suspension; and
(C) a solid-liquid separation step of separating the stabilized suspension of the fine particles into the solid fine particles and the solvent,
wherein in the mixing/precipitation step (A), the poor solvent and the organic compound-containing solution are mixed with each other while irradiating an ultrasonic wave thereto.

10. A process for producing fine particles of an organic compound, comprising continuously mixing a poor solvent in which the organic compound is hardly soluble, with an organic compound-containing solution prepared by dissolving the organic compound in a good solvent which is miscible with the poor solvent and in which the organic compound is readily soluble,
wherein the poor solvent and the organic compound-containing solution are mixed with each other by means of a coaxial mixer having a main flow path and an introduction flow path disposed along a centerline of the main flow path, the poor solvent is passed through the main flow path whereas the organic compound-containing solution is passed through the introduction flow path, and an ultrasonic wave is irradiated to a region where the poor solvent and the organic compound-containing solution are contacted with each other.
